# EUROPÄISCHE PATENTANMELDUNG

(11) **EP 1 170 019 A2**
(43) Veröffentlichungstag der Anmeldung: **09.01.2002**
(21) Anmeldenummer: 01107252.7
(22) Anmeldetag: 23.03.2001
(51) Int. Cl.: A61K 41/00

(54) **Homöopathischen Vorrichtung zur Wiedergabe von homöopathischen Informationen**

(30) Priorität: 06.07.2000 AT 4962000
(71) Anmelder: Quintsysteme für holopathische Medizin Ges.m.b.H., A-3107 St. Pölten (AT)
(72) Erfinder: Dillinger, Klaus, Dipl.-Ing., 3100 St. Pölten (AT); Steiner, Christian, Dr., 9073 Viktring (AT)
(74) Vertreter: Grabherr, Claudia, Dipl.Ing.

(57) **Zusammenfassung**

Homöopathische Information, die durch ein erfindungsgemäßes Verfahren in einem Dateiformat abgespeichert wurde, das zur Übertragung und Wiedergabe von Musik geeignet ist, kann durch eine erfindungsgemäße Vorrichtung wiedergegeben werden, die aus einem herkömmlichen Wiedergabegerät (4, 5, 7, 11) für digital aufgezeichnete Musik und einem Zusatzgerät (6) zum Abgeben von elektromagnetischen Schwingungen besteht. Bei einem Verfahren zur Wiedergabe von homöopathischer Information werden aus einer Vielzahl von auf einem Speichermedium gespeicherten Spektren Spektren ausgewählt und auf ein datenverarbeitendes Gerät (4, 7) geladen, wobei der Zugriff auf das Speichermedium (2) über Internet (3) erfolgt, die Spektren bzw. das Spektrum auf einem Internetsite (1) ausgewählt werden und über das Internet (3) in das datenverarbeitende Gerät (4, 7) geladen werden. Die dazu verwendete Vorrichtung enthält erfindungsgemäß eine Einrichtung zur Datenübertragung aus dem Internet (3) via Modem oder Funk.

## Beschreibung

Die Erfindung betrifft ein Verfahren zur Aufzeichnung von homöopathischen Informationen durch Digitalisierung eines analogen elektromagnetischen Spektrums und Abspeichern des digitalisierten Spektrums auf einem elektronischen Speichermedium einer Datenverarbeitungsanlage.

Die Erfindung betrifft weiters ein Verfahren zur Wiedergabe von homöopathischen Informationen in Form von elektromagnetischen Schwingungen, wobei ein oder mehrere ausgewählte(s) substanz- oder körperspezifische(s) Spektren (Spektrum) aus einer Vielzahl von auf einem Speichermedium gespeicherten Spektren auf ein datenverarbeitendes Gerät geladen werden, die Spektren (das Spektrum) in eine analoges Signal gewandelt werden und verstärkt werden und dann durch ein Gerät zur Abgabe von elektromagnetischen Schwingungen vorzugsweise mindestens eine Elektrode oder Antenne abgegeben werden, und die Verwendung des Verfahrens zur Herstellung von homöopathischen Medikamenten und Testsubstanzen.

Schliesslich betrifft die Erfindung auch eine Vorrichtung zur Wiedergabe von homöopathischen Informationen in Form von elektromagnetischen Schwingungen mit einem Speicher, einem D/A-Konverter und einem Gerät zur Abgabe von elektromagnetischen Schwingungen.

In der AT 130 U ist ein Verfahren beschrieben, mit dem das energetische Spektrum von beliebigen Substanzen aufgezeichnet und wiedergegeben werden kann. Die Aufzeichnung kann dabei entweder analog oder digital erfolgen.

In der AT 1358 U sind mehrere Vorrichtungen und Verfahren beschrieben, mit denen homöopathische Informationen, die in technisch gespeicherter Form vorliegen, auf Personen oder die Umgebung appliziert werden können.

In den erwähnten Gebrauchsmusterregistrierungen ist dokumentiert, dass die Wirkung homöopathischer Medikamente nicht biochemischer, sondern biophysikalischer Natur ist. Als Vermittlungsprinzip werden elektromagnetische Wellen angenommen. Es ist daher möglich, mittels geeigneter Vorrichtungen homöopathische Informationen digital in einer Computeranlage zu speichern, wiederzugeben und auf Personen zu applizieren.

Die Begriffe "homöopathische Information" und "energetische Information" bezeichnen in diesem Dokument den langwelligen Anteil des Schwingungsspektrums substanz- oder körperspezifischer Informationen im Sinne der erwähnten österreichischen Gebrauchsmusterregistrierungen.

Die Verwendung solcher homöopathischer Informationen in technisch gespeicherter Form für die Diagnose und Behandlung von Krankheiten hat den grossen Vorteil gegenüber herkömmlichen homöopathischen Medikamenten und Testsubstanzen, dass sie praktisch unbegrenzt lagerfähig sind, wohingegen herkömmliche homöopathische Medikamente und Testsubstanzen bei langer oder schlechter Lagerung rasch ihre Information und damit ihre Wirkung verlieren.

Nachteil der bisher bekannten Verfahren und Vorrichtungen ist, dass diese bisher nur in einigen wenigen Instituten und Ordinationen Verwendung gefunden haben, da die benötigten Vorrichtungen und die benötigten Daten sehr teuer sind. Dadurch ist auch der Zeitaufwand für eine Behandlung, die über einen grösseren Zeitraum durchgeführt werden muss sehr gross, da der Patient für jede Behandlungseinheit das Institut bzw. die Ordination aufsuchen muss. Natürlich sind damit auch die Behandlungskosten relativ hoch.

Aufgabe der vorliegenden Erfindung ist es, eine Möglichkeit zu finden, die oben genannten Nachteile zu beseitigen und die Technologie der homöopathischer Informationen in technisch gespeicherter Form kostengünstig und an jedem beliebigen Ort nutzbar zu machen.

Die Aufgabe wird dadurch gelöst, dass bei einem Verfahren zur Aufzeichnung von substanzspezifischen energetischen Informationen durch Digitalisierung eines analogen elektromagnetischen Spektrums und Abspeichern des digitalisierten Spektrums auf einem elektronischen Speichermedium einer Datenverarbeitungsanlage, das digitalisierte Spektrum in einem Dateiformat abgespeichert wird, das zur Übertragung und Wiedergabe von Musik geeignet ist und zwar vorzugsweise in einem MP3-Format. Die Verwendung existierender technischer Standards zur Übertragung und Wiedergabe von Musik für das Aufzeichen der substanzspezifischen Informationen erlaubt die Verarbeitung der Spektren mit Programmen und Geräten zur Übertragung und Wiedergabe von Musik. So können diese Spektren über das Internet an jedes Gerät übertragen werden das dieses Dateiformat unterstützt, d.h. an PCs, digitale Musikwiedergabegeräte, Mobilfunktelefone. Das MP3-Format ist im Internet zur Verteilung von Musikstücken weit verbreitet und wird von praktisch allen Web-Endgeräten unterstützt. Es ermöglicht die qualitativ hochwertige Abspeicherung und Wiedergabe von digitalen Schwingungsinformationen bei gleichzeitig maximaler Kompression. Beide Aspekte sind für diese Anwendung wichtig. Es gibt kleine, tragbare MP3-Player auf dem Markt, mit denen diese Schwingungsinformationen wiedergegeben werden können.

Bei einem Verfahren zur Wiedergabe von homöopathischen Informationen in Form von elektromagnetischen Schwingungen, wobei ein oder mehrere ausgewählte(s) homöopathische(s) Spektren (Spektrum) aus einer Vielzahl von auf einem Speichermedium gespeicherten Spektren auf ein datenverarbeitendes Gerät geladen werden, die Spektren (das Spektrum) in eine analoges Signal gewandelt werden und verstärkt werden und dann durch ein Gerät zur Abgabe von elektromagnetischen Schwingungen vorzugsweise mindestens eine Elektrode oder Antenne abgegeben werden, erfolgt erfindungsgemäss der Zugriff auf das Speichermedium über Internet, die Spektren bzw. das Spektrum werden auf einem Internetsite ausgewählt und über das Internet in das datenverarbeitende Gerät geladen. Homöopathische Informationen können durch dieses Verfahren im Internet angeboten und abgerufen werden. Dadurch wird eine mobile Diagnose und Therapie möglich, wobei die jeweils benötigten Testsubstanzen und Therapeutika aus dem Internet geladen werden.

Vorzugsweise erfolgt die Auswahl der Spektren durch Angabe einer oder mehrere Krankheitssymptome oder der Indikation(en) um die Verwendung der angebotenen Informationen zu erleichtern. So können fertig zusammengestellter homöopathische Rezepte auf einem Web-Site, gegliedert nach Indikationen (z.B Abgespanntheit, Verspannungen, Prämenstruelles Syndrom, Verdauungsstörungen, Kopfschmerzen, Erkältung, Heuschnupfen,...) zur Verfügung gestellt werden. Und es besteht die Möglichkeit des Downloadings per Mausklick auf ein Endgerät des Kunden.

Gemäß einer besonderen Ausführungsform des erfindungsgemässen Verfahrens wird nach der Auswahl der Spektren die Summe der Spektren errechnet und an das datenverarbeitende Gerät übertragen.

Erfindungsgemäß kann zu jedem Spektrum bzw. zu jeder Spektrensumme eine Dosierinformation, d.h. eine Information über die Dauer, die Zahl und den zeitlichen Abstand der Wiederholungen der Abgabe durch das Gerät zur Abgabe von elektromagnetischen Schwingungen mitübertragen wird, wobei die Dosierinformation vorzugsweise auch von den angegebenen Symptomen oder Indikation(en) abhängig ist, so dass eine sichere und richtige Dosierung gewährleistet ist. Dies erlaubt eine Wiedergabe von Rezepten in bestimmten, einstellbaren Zeitintervallen und -dauern. Dazu können z.B. die eingebauten Programmiermöglichkeiten von MP3-Geräten eingesetzt werden. Wie lange und wie oft die Rezepte für die Therapie wiedergegeben werden sollen, hängt von der Indikation ab. Die Einstellungsinformation für das Wiedergabegerät erhält der Anwender zusammen mit der Schwingungsinformation.

Die Zusammenstellung sequenzieller Therapieprogramme aus mehreren Rezepten in beliebiger Kombination und Abfolge ist somit möglich.

Das Verfahren kann erfindungsgemäß auch zur Herstellung von homöopathischen Medikamenten und Testsubstanzen verwendet werden, wobei eine Trägersubstanz, wie insbesondere eine Flüssigkeit, eine Salbengrundlage oder Globuli in die unmittelbare Umgebung des Gerätes zur Abgabe von elektromagnetischen Schwingungen gebracht wird. Dadurch wird die homöopathische Information auf die Trägersubstanz aufgeprägt und sozusagen ein künstliches Homöopathikum hergestellt.

Die erfindungsgemässe Vorrichtung zur Wiedergabe von homöopathischen Informationen in Form von elektromagnetischen Schwingungen mit einem Speicher, einem D/A-Konverter und einem Gerät zur Abgabe von elektromagnetischen Schwingungen, besteht aus einem herkömmlichen Wiedergabegerät für digital aufgezeichnete Musik und einem Zusatzgerät zum Abgeben elektromagnetischer Schwingungen. Solche Wiedergabegeräte werden in absehbarer Zeit in fast jedem Haushalt vorhanden sein, in Form eines PCs oder auch eines tragbaren Players. Die Kosten dafür sind weit geringer als die Vorrichtungen gemäss den österreichischen Gebrauchsmustern A 130 U und A 1358 U. Denkbar wäre auch,dass solche tragbaren Geräte als Leihgeräte in Apotheken oder bei anderen Stellen erhältlich sind. Auch das Zusatzgerät ist kostengünstig herzustellen.

Vorzugsweise wird das Zusatzgerät am Analogausgang des herkömmlichen Wiedergabegerätes für digital aufgezeichnete Musik angeschlossen. Mithilfe solcher spezieller Zusatzgeräte, die an die Kopfhörerbuchse gängiger Wiedergabegeräte angeschlossen werden können, wird eine effiziente, benutzerfreundliche und kostengünstige Applikation der übertragenen homöopathischen Informationen ermöglicht.

Z.B. kann ein solches herkömmliches Wiedergabegerät für digital aufgezeichnete Musik ein MP3-Player sein, wie er als kleines tragbares Gerät heute bereits am Markt erhältlich ist.

Vorzugsweise enthält die Vorrichtung eine Einrichtung zur Datenübertragung aus dem Internet via Modem oder Funk, sodass die anzuwendenden Spektren durch diese Einrichtung in die Vorrichtung übertragen werden können.

Z.B. kann das herkömmliche Wiedergabegerät für digital aufgezeichnete Musik ein internetfähiges Mobilfunktelefon sein, das das Dateiformat zur Musikwiedergabe unterstützt. Dieses kann z.B. mittels WAP-Standard direkt an das Internet angeschlossen werden. Ein Gerät also, das die zu behandelnde Person sowieso bei sich trägt.

Gemäß einer bevorzugten Ausführungsform ist das Zusatzgerät ein Gerät, das ein schwaches elekromagnetisches Feld erzeugt, dem die substanzspezifischen Schwingungen aufmoduliert sind. Aufgrund von Erfahrungen in der energetischen Medizin ist bekannt, dass bereits ein derartiges schwaches Feld ausreicht, um physiologische Wirkungen zu erzielen.

Das Zusatzgerät kann aber auch ein herkömmliches Magnetfeldtherapie- oder ein Magnetmattengerät sein.

Unabhängig vom Dateiformat für die Spektren enthält eine erfindungsgemässe Vorrichtung zur Wiedergabe von homöopathischen Informationen in Form von elektromagnetischen Schwingungen mit einem Speicher, einem D/A-Konverter und einem Gerät zur Abgabe von elektromagnetischen Schwingungen, eine Einrichtung zur Datenübertragung aus dem Internet via Modem oder Funk, sodass die anzuwendenden Spektren durch diese Einrichtung in die Vorrichtung übertragen werden können.

Vorzugsweise enthält die Vorrichtung Einrichtungen zum Messen physiologischer Parameter von Lebewesen. Damit wird ein System für die Selbst-Diagnose geschaffen, das einer Kombination der zuvor beschriebenen Vorrichtung zur Wiedergabe von homöopathischen Informationen mit einem handelsüblichen, einfachen Testgerät, mit dem physiologische Parameter (zB Haut-Leitwert, Pulsfrequenz und -form, EEG, etc.) technisch erfasst werden können, entspricht.

Solche Testgeräte sind beispielsweise aus Biofeedback-Systemen bekannt. Ein Anwender kann nun aus dem Internet Testsubstanzen im MP3-Format laden und mittels des Testgeräts feststellen, wie er darauf reagiert. Damit wird Individualmedizin in Selbstbedienung über das Internet möglich. Das Testprinzip beruht auf der Beobachtung, dass "unpassende" Substanzen beim Anwender Stress erzeugen, der mit dem Biofeedback-Testgerät sichtbar gemacht werden kann. Umgekehrt bewirken positive Substanzen einen Energieausgleich, der sich ebenfalls in charakteristisch veränderten Biofeedback-Parametern niederschlägt.

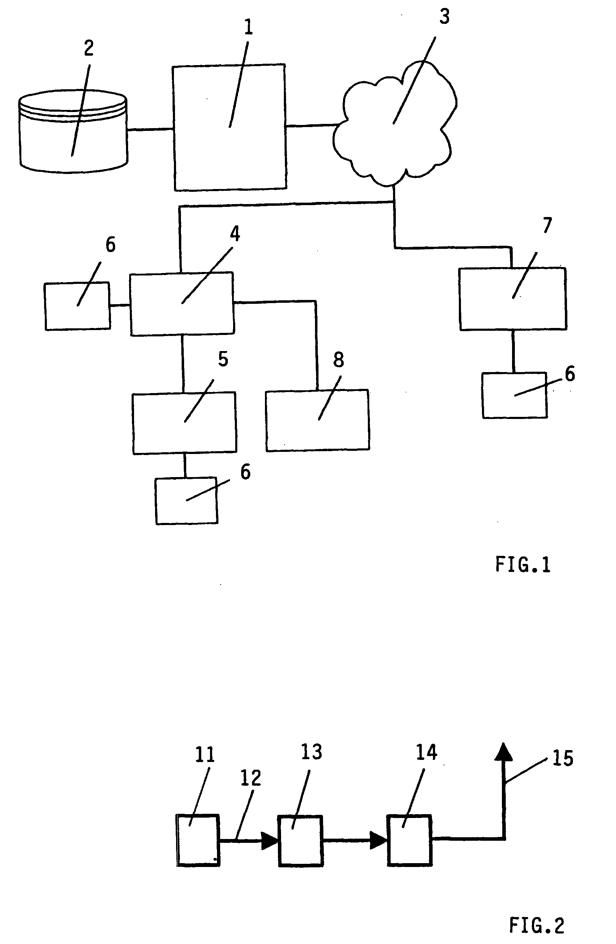

Im Folgenden soll die Erfindung noch anhand der in den Figuren 1 und 2 dargestellten Schemata erklärt werden.

Die Fig. 1 gibt einen Überblick über die Struktur des erfindungsgemässen Systems. Die Fig.2 zeigt das Prinzip der Vorrichtung zur Wiedergabe homöopathischen Informationen.

Für die technische Implementierung des Verfahrens wird ein Internetsite 1 installiert, auf dem die gewünschten homöopathischen Informationen in einer Datenbank 2 gespeichert sind. Diese Informationen können grundsätzlich in jedem Datenformat gespeichert sein, das eine Darstellung von Schwingungsinformationen in der geforderten hohen Qualität erlaubt. Aus praktischen Gründen wird man sich dabei aber eines der im Internet verbreiteten Formate für die digitale Speicherung von Musik bedienen. Zum einen bringt die technische Implementierung eines Audio-HiFi-Systems erfahrungsgemäss auch sehr gute Resultate bei der Verarbeitung homöopathischer Schwingungen, zum anderen gibt es eine Fülle von Hard- und Software zur Unterstützung der Internet-Musikstandards auf dem Markt. Aus heutiger Sicht bietet sich dazu vor allem das MP3-Format an.

Die Abspeicherung der homöopathischen Informationen in der Internet-Datenbank erfolgt durch den Anbieter. Es können sowohl einzelne Substanzen als auch komplexe Mischungen mehrerer Substanzen in unterschiedlichen homöopathischen Potenzen abgespeichert werden. Die Art der abzuspeichernden Substanzen ist durch die jeweilige Anwendung bestimmt und unterliegt grundsätzlich keinerlei Einschränkungen.

Um Substanzen für Testung oder Therapie zu applizieren nimmt der Anwender über das Internet 3 Kontakt mit der Substanz-Datenbank 2 auf. Er überträgt die gewünschten Substanzen auf sein Endgerät. Als Endgeräte kommen grundsätzlich sämtliche an das Internet anschliessbare Geräte (sogenannte Internet-Appliances) in Betracht. Es ist dabei nicht zwingend notwendig, dass das Endgerät selbst das Datenformat für die gespeichertern Substanzinformationen wiedergeben kann. Es muss lediglich in der Lage sein, die heruntergeladenen Dateien zu speichern und in ein geeignetes Wiedergabegerät zu übertragen. In Fig.1 werden als Endgeräte ein PC 4 bzw ein Mobiltelefon 7 mit integrierter MP3-Wiedergabemöglichkeit eingesetzt. Aus dem PC können die heruntergeladenen Dateien auf Wunsch weiter in ein tragbares Wiedergabegerät (MP3-Player 5) übertragen werden.

Die Applikation der übertragenen Schwingungsdaten aus dem Wiedergabegerät geschieht mittels eines erfindungsgemässen Zusatzgeräts 6, dem sog. Applikator, das vorzugsweise an den Kopfhörerausgang des Wiedergabegeräts oder des PC angeschlossen wird. Dieses Gerät ist gemäss Prinzipien aus AT 1358 U wie in Fig.2 gezeigt aufgebaut.

Das Wiedergabegerät 11 erzeugt aus den digital übertragenen Dateien analoge Substanzinformationen, welche vorzugsweise über den Kopfhörerausgang 12 abgegriffen und von einem Verstärker 13 verstärkt werden. Der Verstärker ist mit einem Sender 14 verbunden, welcher über eine Antenne 15 ein elektromagnetisches Feld abstrahlt. Dieses Feld enthält die gewünschten homöopathischen Informationen. Je nach Verwendungszweck der Vorrichtung kann die Antenne 15 in unterschiedlichen Bauarten ausgeführt sein. Zweckmässigerweise wird dieses Gerät in kompakter Bauweise und mit Stromversorgung über Batterien ausgeführt. Ein solcher Applikator könnte auch als integrierte Schaltung ausgeführt werden. Das ermöglicht es, Wiedergabegeräte bereits serienmässig durch den Hersteller mit Applikatoren auszurüsten.

Eine andere Form des Applikators erlaubt die Aufprägung der homöopathischen Information auf stoffliche Medien, wie im AT 1358 U beschrieben.

Parallel zur Kombination Wiedergabegerät / Applikator und unabhängig davon kann ein handelsübliches Testgerät für physioliogische Parameter 8 eingesetzt werden. Der Anwender kann so unabhängig von indikationsgebundenen Rezepten durch Testung bestimmen, welches der verfügbaren abgespeicherten homöopathischen Rezepte sich für ihn am besten eignet.

Statt mittels des beschriebenen Applikators können die Substanzschwingungen auch mittels konventioneller (Klebe-)Elektroden appliziert werden. In diesem Fall muss ein speziell konstruiertes Interface-Kabel verwendet werden, um Schäden an der Elektronik des Wiedergabegeräts zu vermeiden.

## Patentansprüche

1. Verfahren zur Aufzeichnung von homöopathischen Informationen durch Digitalisierung eines analogen elektromagnetischen Spektrums und Abspeichern des digitalisierten Spektrums auf einem elektronischen Speichermedium einer Datenverarbeitungsanlage, **dadurch gekennzeichnet, daß** das digitalisierte Spektrum in einem Dateiformat abgespeichert wird, das zur Übertragung und Wiedergabe von Musik geeignet ist.

2. Verfahren nach Anspruch 1, **dadurch gekennzeichnet, daß** das Dateiformat ein MP3-Format ist.

3. Verfahren zur Wiedergabe von homöopathischen Informationen in Form von elektromagnetischen Schwingungen, wobei ein oder mehrere ausgewählte(s) homöopathische(s) Spektren (Spektrum) aus einer Vielzahl von auf einem Speichermedium gespeicherten Spektren auf ein datenverarbeitendes Gerät geladen werden, die Spektren (das Spektrum) in ein analoges Signal gewandelt und verstärkt werden und dann durch ein Gerät zur Abgabe von elektromagnetischen Schwingungen, vorzugsweise eine Elektrode oder Antenne abgegeben werden, **dadurch gekennzeichnet, daß** der Zugriff auf das Speichermedium (2) über Internet (3) erfolgt, die Spektren bzw. das Spektrum auf einem Internetsite (1) ausgewählt werden (wird) und über das Internet (3) in das datenverarbeitende Gerät (4,7) geladen werden (wird).

4. Verfahren nach Anspruch 3, **dadurch gekennzeichnet, daß** die Auswahl der Spektren durch Angabe einer oder mehrerer Krankheitssymptome oder der Indikation(en) erfolgt.

5. Verfahren nach Anspruch 3 oder 4, **dadurch gekennzeichnet, daß** nach der Auswahl der Spektren die Summe der Spektren errechnet wird und an das datenverarbeitende Gerät (4, 7) übertragen wird.

6. Verfahren nach Anspruch 4 oder 5, **dadurch gekennzeichnet, daß** zu jedem Spektrum bzw. zu jeder Spektrensumme eine Dosierinformation, d.h. eine Information über die Dauer, die Zahl und den zeitlichen Abstand der Wiederholungen der Abgabe durch das Gerät (6, 8) zur Abgabe von elektromagnetischen Schwingungen, mitübertragen wird, wobei die Dosierinformation vorzugsweise auch von den angegebenen Symptomen oder Indikation(en) abhängig ist.

7. Verwendung des Verfahrens nach einem der Ansprüche 3 bis 6 zur Herstellung von homöopathischen Medikamenten und Testsubstanzen, **dadurch gekennzeichnet, daß** eine Trägersubstanz, wie insbesondere eine Flüssigkeit, eine Salbengrundlage oder Globuli in die unmittelbare Umgebung des Gerätes (6, 8) zur Abgabe von elektromagnetischen Schwingungen gebracht wird.

8. Vorrichtung zur Wiedergabe von homöopathischen Informationen in Form von elektromagnetischen Schwingungen mit einem Speicher, einem D/A-Konverter und einem Gerät zur Abgabe von elektromagnetischen Schwingungen, **dadurch gekennzeichnet, daß** die Vorrichtung aus einem herkömmlichen Wiedergabegerät (4, 5, 7, 11) für digital aufgezeichnete Musik und einem Zusatzgerät (6) zum Abgeben von elektromagnetischen Schwingungen besteht.

9. Vorrichtung nach Anspruch 8, **dadurch gekennzeichnet, daß** das Zusatzgerät (6) am Analogausgang des herkömmlichen Wiedergabegerätes (4, 5, 7, 11) für digital aufgezeichnete Musik angeschlossen ist.

10. Vorrichtung nach Anspruch 8 oder 9, **dadurch gekennzeichnet, daß** das herkömmliche Wiedergabegerät (4, 5, 7, 11) für digital aufgezeichnete Musik ein MP3-Player ist.

11. Vorrichtung nach einem der Ansprüche 8 bis 10, **dadurch gekennzeichnet, daß** die Vorrichtung eine Einrichtung zur Datenübertragung aus dem Internet (3) via Modem oder Funk enthält.

12. Vorrichtung nach Anspruch 11, **dadurch gekennzeichnet, daß** das herkömmliche Wiedergabegerät für digital aufgezeichnete Musik ein internetfähiges Mobilfunktelefon (7) ist, das Dateiformat zur Musikwiedergabe unterstützt.

13. Vorrichtung nach einem der Ansprüche 8 bis 12, **dadurch gekennzeichnet, daß** das Zusatzgerät (6) ein Gerät ist, das ein schwaches elekromagnetisches Feld erzeugt, dem die substanzspezifischen Schwingungen aufmoduliert sind.

14. Vorrichtung nach einem der Ansprüche 8 bis 12, **dadurch gekennzeichnet, daß** das Zusatzgerät ein Magnetfeldtherapie- oder ein Magnetmattengerät ist.

15. Vorrichtung zur Wiedergabe von homöopathischen Informationen in Form von elektromagnetischen Schwingungen mit einem Speicher, einem D/A-Konverter und einem Gerät zur Abgabe von elektromagnetischen Schwingungen, **dadurch gekennzeichnet, daß** die Vorrichtung eine Einrichtung zur Datenübertragung aus dem Internet (3) via Modem oder Funk enthält.

16. Vorrichtung nach Anspruch 15, **dadurch gekennzeichnet, daß** die Vorrichtung Einrichtungen (8) zum Messen physiologischer Parameter von Lebewesen enthält.
